(19) Europäisches Patentamt
European Patent Office
Office européen des brevets

(11) **EP 3 511 847 A1**

(12) **EUROPEAN PATENT APPLICATION**
published in accordance with Art. 153(4) EPC

(43) Date of publication:
**17.07.2019 Bulletin 2019/29**

(51) Int Cl.:
*G06F 19/24* (2011.01)  *G06F 19/28* (2011.01)
*G06F 19/22* (2011.01)

(21) Application number: **17886616.6**

(22) Date of filing: **22.12.2017**

(86) International application number:
**PCT/KR2017/015377**

(87) International publication number:
**WO 2018/124661 (05.07.2018 Gazette 2018/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **27.12.2016 KR 20160180336**

(71) Applicant: **Chunlab, Inc.**
**Seoul 06725 (KR)**

(72) Inventors:
• **YOON, Seok-Hwan**
**Seoul 08848 (KR)**
• **KIM, Taewook**
**Seongnam-si**
**Gyeonggi-do 13550 (KR)**

(74) Representative: **Agasse, Stéphane et al**
**Cabinet GERMAIN & MAUREAU**
**B.P. 6153**
**69466 Lyon Cedex 06 (FR)**

(54) **METHOD FOR OBTAINING RIBOSOMAL RNA (RRNA) SEQUENCE INFORMATION AND METHOD FOR IDENTIFYING MICROORGANISM USING RRNA**

(57)    The present disclosure relates to a method for obtaining rRNA sequence information, comprising the steps of calling an rRNA sequence from full-length genomic DNA sequence information, assembling the rDNA to form rRNA contigs; and extracting 16S rRNA having high completeness from the rDNA contigs and correcting an assembly error, and a method for identifying a microorganism, using the rDNA information.

FIG. 2

**Description**

## BACKGROUND OF THE INVENTION

Field of the invention

The present invention relates to a method for obtaining a ribosomal RNA sequence information and a method for identifying a microorganism using the ribosomal RNA sequence information and, more particularly, to a method for obtaining a ribosomal RNA (rRNA) sequence information comrpising acquiring genome NGS reads including a ribosomal DNA (rDNA) of a single organism by using the next generation sequencing (NGS), selecting reads including rDNA sequence information from the genome NGS reads, segmneting the reads and assembling the segmentes.

Description of the Prior Art

Culture methods have been traditionally used for identification of specific microorganisms, but over the last two decades, culture-independent molecular biology methods that allow identification without use of media have been increasingly developed, making a great contribution not only to microbial identification but also to research into microbial diversity in various different habitats. Particularly, the DNA sequencing method developed by Sanger at al. (1977) allowed studies on microflora in the early stage. In the Sanger sequencing method, microbial 16S rRNA is amplified, cloned, and sequenced to identify microbial flora. Although the Sanger sequencing approach has revealed DNA sequences and greatly contributed to advances in molecular biology, technical difficulties, high cost, and much time associated therewith make it difficult to simultaneously analyze a large number of clones from a large number of samples. Thus, researchers have difficulty in applying the Sanger method to studies on microbial ecology. The Sanger method requires much time and is limited by a number of samples that are simultaneously analyzable.

As alternatives to the Sanger method, new methods called next-generation DNA sequencing have been introduced. The new methods differ from one to another in the length of sequence reads and throughput and find applications in versatile bioscience fields.

In this regard, the analysis targets include nucleotide sequences in ribosomal RNA (rRNA) and especially in the small subunit (16S rRNA) of bacteria and archaea. 16S rRNA genes have advantages over other genes as follows: first, the highly conserved regions are used for designing PCR primers; second, hypervariable regions allow the accurate taxonomic and phylogenetic identification of members in family clusters thereof; third, horizontal gene transfer is hardly found among classification groups; and lastly, data accumulated for a wide range of 16S rRNA nucleotide sequences allows accurate classification and is highly advantageous for diversity analysis. For fungi, 18S rRNA genes or internal transcribed spacer (ITS) regions are used as markers for species identification markers.

Various next-generation sequencing approaches produce massive nucleotide sequence information from the full-length genomic DNA of a target microorganism. Through the assembly of sequence reads, the extraction of specific nucleotide sequence information, and the analysis of the extracted nucleotide sequence information, a target gene to be analyzed, for example, a 16S rRNA sequence can be applied to a pre-existing data pool to identify a microorganism to be analyzed. However, the assembly process using read data requires a great deal of time due to massive data, complicated data structure and processing, etc. Another problem with the assembly process is that because segmentation into short sequences (k-mer) is conducted for effective assembly, such algorithmic limitations are apt to omit information of segment sequences less than a certain size, making it difficult to acquire information on the entire region of 16S rRNA gene.

## SUMMARY OF THE INVENTION

An embodiment of the present disclosure provides a method for obtaining rRNA sequence information, for example, rRNA sequence information with a high completeness, by using genome NGS reads inclusive of ribosomal DNA (rDNA) sequence information of a single organism obtained by next generation sequencing (NGS).

Another embodiment of the present disclosure provides a method for identifying a microorganism using full-length rRNA sequence information, for example, rRNA sequence information with a high completeness, the method comprising aligning the rRNA, for example, 16S rRNA sequence information with a 16S rRNA database.

A further embodiment of the present disclosure provides a computer reading method for obtaining full-length rRNA sequence information, using rRNA sequence information in nucleotide analysis method for an experiment sample.

An additional embodiment of the present disclosure provides a computer executable instruction, stored in a computer readable storage medium, for executing a method for obtaining full-length rRNA sequence information using rRNA sequence information in nucleotide analysis method for an experiment sample, or a computer readable storage medium (or computer recordable medium) storing the computer executable instruction.

**BRIEF DESCRIPTION OF THE DRAWINGS**

The above and other objects, features and advantages of the present invention will be more apparent from the following detailed description taken in conjunction with the accompanying drawings, in which:

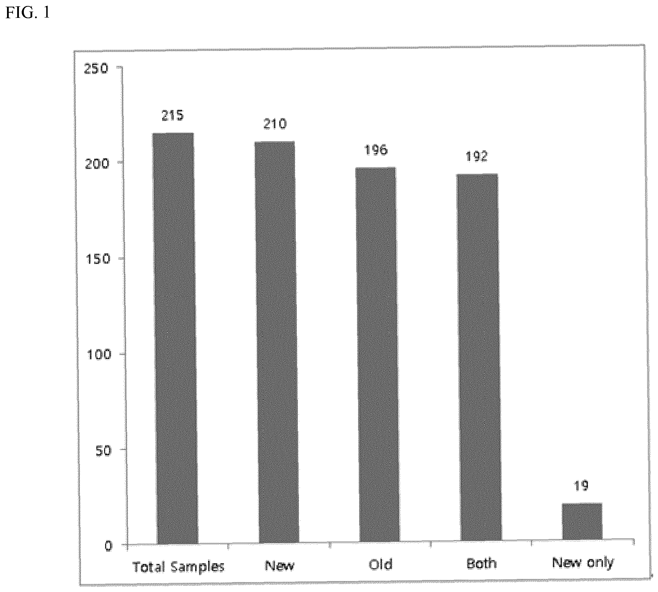

FIG. 1 is a view depicting counts of samples in which full-length 16S rRNA sequences were called using a convention method and a method according to an embodiment of the present disclosure, wherein "New" stands for the use of the method according to an embodiment of the present disclosure, "Old" for the use of a conventional method, "Both" for the use of both the conventional method and the method of the present disclosure, and "New only" for the method according to an embodiment of the present disclosure alone, in successfully calling full-length 16S rRNA sequences; and

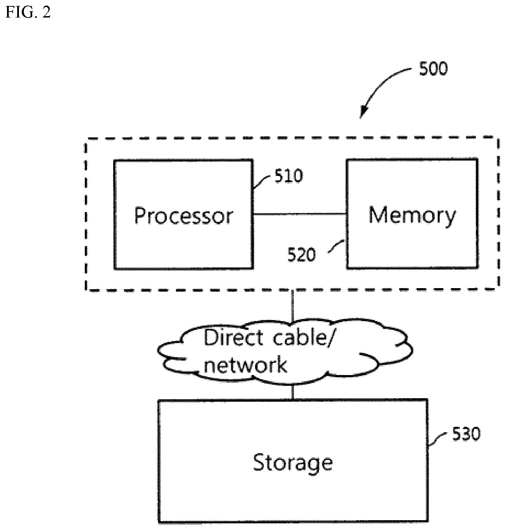

FIG. 2 is an illustration of a computer-readable medium according to an embodiment of the present disclosure.

**DETAILED DESCRIPTION OF THE EXEMPLARY EMBODIMENTS**

An embodiment of the present disclosure provides a method for obtaining a ribosomal DNA or ribosomal RNA sequence information with a high completeness of a single organism, comprising the steps of: acquiring genome NGS reads of a single organism; calling reads including rDNA sequence information out of the genome NGS reads; assembling the called reads; and determining and excising rDNA sequence regions in rDNA contigs to obtain rDNA sequence regions. The rDNA may be 16S rDNA, 23S rDNA, or 5S rDNA, but is not limited thereto.

A particular embodiment of the present disclosure relates to a method for obtaining a ribosomal DNA having a completeness of 0.90 or higher in a single organism, the method comprising the steps of:

(a) obtaining genome NGS reads including ribosomal DNA (rDNA) in a single organism with next generation sequencing (NGS),
(b) selecting the reads including rDNA sequence information from the genome NGS reads,
(c) segmenting the selected reads and merging the segments for assembly to form one or more rDNA contigs, and
(d) determining and excising an rDNA sequence region in the one or more rDNA contigs, to obtain the rDNA sequence region.

Another embodiment of the present disclosure pertains to a method for obtaining full-length ribosomal RNA sequence information of a single organism from the genome sequence information of the single organism which includes full-length rDNA sequence information acquired using a sequencing assay with a computer device operating with at least one processor, the method comprising:

acquiring genome NGS reads including full-length rDNA sequence information of a single organism by next generation sequencing (NGS);
primarily selecting rDNA sequence information from the genome NGS reads of the single organism to afford entire sequences of reads which include sequence regions of rDNA region and are in a length of a K-mer value or longer, for assembly;
segmenting the selected reads into segments with a K-mer value of 7 to 36 and assembling the segments to form an rDNA contig; and
secondarily selecting by excising a full-length rDNA sequence region out of the rDNA contigs to obtain a full-length rDNA sequence.

The secondary selecting step in which regions other than the rDNA sequence region are cut out to leave the rDNA sequence region may be to correct at least one selected from the group consisting of a nucleotide sequence error, a false gap, a tandem repeat error, a monopolymer error, and a single nucleotide polymorphism error which may be gernerated in the mergng step of segments. In addition, the method for obtaining full-length rRNA sequence information using a nucleotide sequencing assay may further comprise correcting an assembly error of the secondarily selected rDNA sequence information.

The present disclosure relates to a method for identifying a microorganism, using an rRNA nucleotide sequencing assay, the method comprising: obtaining ribosomal DNA having a completeness of 0.90 or higher in a single organism, and comparing sequence information of the obtained rRNA with a taxonomic database of rRNAs to identify a microorganism.

An additional embodiment of the present disclosure pertains to a computer reading method for analyzing full-length rRNA sequence information using a nucleotide sequence assay, the method comprising the step of obtaining rRNA sequence information having a completeness of 0.90 or higher by means of a nucleotide sequencing assay.

Contemplated according to a further embodiment of the present disclosure is a storage medium which records computer readable instruction for allowing the execution of the step of obtaining rRNA sequence information having a completeness of 0.90 or higher by means of a nucleotide sequencing assay.

The present disclosure will be described below in more detail.

The method for obtaining rDNA sequence information of high completeness in accordance with the prsent disclosure comprises the following steps of:

(a) acquiring genome NGS reads including ribosomal DNA (rDNA) sequence information by the next generation sequencing (NGS),

(b) selecting the reads including rDNA sequence information from the genome NGS reads,

(c) segmenting the selected reads and merging the segments to form at least one rDNA contig for assembly

(d) determining and excising an rDNA sequence region out of the at least one rDNA config.

In the present disclosure, the step (a) of acquiring genome NGS reads of a single organism is carried out by massively parallel DNA sequencing in which genomic DNA is cut into a large number of segments from each of which sequence reads are then obtained, followed by merging the reads by means of various bioinformatics approaches to sequence the genome.

In a conventional method of obtaining rRNA sequence information, de novo assembly of the entire genome including information of all genes is conducted to give data from which putative 16S rRNA sequence regions are then extracted to obtain full-length rRNA. Such conventional methods are disadvantageous in that the nucleotide sequence of rRNA is partially lost during the de novo genome assembly and full-length rRNA calling which causes a failure to obtain accurate full-length rRNA sequence information, or short rRNA sequence information on only a part of rRNA gene is obtained so that errors are frequently found in results of microorganism identification.

In contrast, the present disclosure concerns a method in which selection is not made of 16S rRNA sequence segments from the data obtained by gene assembly, but of reads (segments) including rRNA gene sequences from genome sequence reads, followed by merging the selected reads, whereby an accurate full-length 16S rRNA gene sequence inclusive of the entire region of rRNA gene can be called.

The method according to the present disclosure can secure the sequence length completeness of rRNA and aims for rDNA sequence information with a completeness of 0.9 or higher, 0.93 or higher, or 0.95 or higher, in consideration of the taxonomic purpose for microbial identification. The length of a bacterial rDNA sequence, although differing from one species to another, generally ranges from about 1,450 bp to 1,500 bp for 16S rRNA. A minimal length corresponding to a completeness of 90 % or higher in a query rDNA may be about at least 1,300 bp or at least 1,450 bp. Considering the full-length rDNA sequence of bacteria, the length of a query rDNA sequence obtained according to the present disclosure may range from about 1,300 bp to 1,800 bp or from about 1,450 bp to 1,800 bp.

As used herein, the term "completeness" refers to an objective measure of the degree of coverage of a query rRNA gene sequence with respect to the full-length, complete rRNA gene sequence. Completeness is defined as the following mathematical formula, wherein completeness values may be expressed as ratios according to the following mathematical formula or as percentages (%):

[Mathematical Formula 1]

$$\text{Completeness} = \frac{L}{C}$$

where L is the length of a query sequence and C is the length of the most similar sequence that is regarded as complete.

The most similar sequence in the database of complete rDNA sequences is identified by using already known rDNA databases. For example, sequences which each include regions covering up to universal primers (27F and 1492R) of 16S rRNA sequence are separately collected from the entire 16S rRNA sequence database to construct a database of complete 16S rRNA sequences, and a query sequence is matched and aligned with the database sequences to detect how many nucleotides of the reference sequence are deleted compared to the database sequences to calculate the completeness.

When algorithms such as Hmmsearch or CMSearch are employed, C may be a length of the profile obtained from sequence pattern information of the database. Over a conventional method in which a region of 16S rRNA sequence information is called from the data obtained after de novo genome assembly to acquire a full-length 16S rRNA sequence, the method for obtaining full-length 16S rRNA sequence information according to the present invention has outstanding advantages in terms of the number of called full-length 16S rRNA samples, the calling integrity of 16S rRNA sequences, the ratio of called full-length 16S rRNA samples, and the loss of 16S rRNA sequence information at the assembly step.

The method for obtaining rRNA sequence information having high completeness using a sequencing assay in accordance with the present disclosure is explained in detail with respect to the steps thereof.

**(a) Step of obtaining genome NGS reads of a single organism**

In the present disclosure, first, genome-wide NGS reads of an organism, obtained by means of next-generation sequencing (NGS), may be provided.

In the present disclosure, the genome-wide NGS reads of an organism may be obtained with NGS, for example, massively parallel sequencing. In the context of the sequence information, polynucleotide segments are reads used for NGS, a number of the polynucleotide segments is a read count or read depth, and an average number of the polynucleotide segments are an average read number.

As used herein, the term "massively parallel sequencing" is a generic name of high-throughput approaches to DNA sequencing in which a genome is randomly digested to numerous polynucleotide segments (reads) which are then simultaneously sequenced, and the sequence reads thus obtained are assembled using bioinformatics to decrypt massive genomic information.

The sequencing may be done using the next-generation sequencing such as that achievable on the 454 platform (Margulies, et al., Nature (2005) 437:376~380), Illumina Genome Analyzer (or Solexa™ platform), Illumina HiSeq2000, HisSeq2500, MiSeq, NextSeq500, Life Tech Ion PGM, Ion Proton, Ion S5, Ion S5XL, SOLiD (Applied Biosystems) or the Helicos True Single Molecule DNA sequencing technology (Harris, et al., Science (2008) 320:106-109), and/or single molecule, real-time (SMRT™) sequencing technology from Pacific Biosciences. In addition, the nanopore sequencing (Soni and Meller, Clin Chem (2007) 53:1996~2001) allows the sequencing of many nucleic acid molecules isolated from a sample at high orders of multiplexing in a parallel fashion (Dear, Brief Funct Genomic Proteomic (2003) 1:397-416). Each of these platforms sequences clonally expanded or non-amplified single molecules of nucleic acid segments. Sequence information of polynucleotide segments can be obtained using a commercially available thermal cycler. In addition, sequencing may be conducted with various other well-known sequencing technologies or modifications thereof.

The genome-wide DNA sequence information of a sample may be directly analyzed using next-generation sequencing or may be genetic information stored in a computer storage medium. Sequence data may be searched for in and downloaded from the Sequence Read Archive of the National Center for Biotechnology Information (NCBI) with the aid of the SRA toolkit program under SRA accession, but is not limited thereto.

Examples of the ribosomal RNA (rRNA) to be obtained by sequencing according to the present disclosure include 16S rDNA, 5S rDNA, and 23S rDNA. 16S rRNA accounts for the 30S small subunit of a prokaryotic ribosome, which is approximately 1,500 bp long. Particularly prokaryotic species share similarity across full 16S rRNA gene and have respective characteristic nucleotide sequence regions which can be used for microbial identification.

**(b) Step of selecting the reads containing rDNA sequence information from the genome NGS reads**

In one embodiment of the present disclosure, the step of selecting rRNA sequence information from the genome NGS reads, for example, the step of selecting reads contaiing rDNA sequence information may be conducted.

In the method of the present disclosure, 16S rRNA sequence segments are not called out of the data obtained by gene assembling all genome NGS reads, but reads (segments) including rRNA gene sequences are first called or extracted out of genome sequence reads, followed by merging the selected reads, whereby an accurate full-length 16S rRNA gene sequence inclusive of the entire region of rRNA gene can be called. Over a conventional method in which a region of 16S rRNA sequence information is called from the data obtained after de novo genome assembly to acquire a full-length 16S rRNA sequence, the method for obtaining full-length 16S rRNA sequence information according to the present invention has outstanding advantages in terms of the number of called full-length 16S rRNA samples, the calling integrity of 16S rRNA sequences, the ratio of called full-length 16S rRNA samples, the loss of 16S rRNA sequence information at the assembly step.

The program with which rDNA sequences are called or NGS reads inclusive of rDNA sequence information are called out of genome NGS reads may be one selected from the group consisting of CMsearch, Hmmsearch, BLAST, and Usearch, with preference for CMsearch, Hmmsearch, and BLAST. The calling may be carried out by calling all reads inclusive of rRNA sequence information or excising only rRNA regions out of the reads. Preferably, rRNA sequence information-bearing reads themselves are called in the calling step.

In the calling step, sequence pattern information obtained by aligning well-known full-length rRNA sequences may be used to call reads having an E-value of $1 \times 10^{-10}$ or $1 \times 10^{-20}$ or less.

With reference to 16S rRNA, the called reads are sequences having regions significantly similar to sequence pattern information which is obtained by applying a hidden Markov model or Covariance model at an E-value of, for example, $1\times10^{-10}$ or less or $1\times10^{-20}$ ($1e^{-20}$) or less to 16S rRNA sequence information or 16S rRNA sequence database by means of respective programs, and can include reads having K-mer long enough to be used for assembly..

In the read calling step, selection is made of the entire reads that are matched with the Hidden Markov Model (HMM) or Covariance Model (CM) profile obtained by alignment with well-known complete 16S rRNA and have a specific E-value or less.

### (c) Step of segmenting selected reads and merging the segments for assembly to form one or more rDNA contigs

In one embodiment of the present disclosure, sequence information of the selected rRNA reads is assembled to form an rRNA contig with a K-mer value of 7 to 36.

In contrast to a conventional method in which full-length rRNA is obtained by selecting rRNA sequence information regions out of the data constructed after de novo genome assembly, the method of the present disclosure states to call reads (segments) inclusive rRNA gene sequences out of genome sequence reads, followed by conducting assembly of the called reads. According to the present disclosure, accurate full-length rRNA gene sequences covering all rRNA gene regions can be obtained.

The step in which the segments are merged to form an rDNA contig may comprise a merging process of segments and a verifying process in which the merged segments are aligned with the called reads to examine whether the merged segments are sequence regions within the reads. Until the formation of the contig is completed, the merging process and the verifying process may be repeated two or more times.

The contigs which are obtained by assembly after calling of rRNA in the present disclosure may be fewer as well as shorter than the contigs obtained by de novo genome assembly. For example, contigs formed according to the present disclosure may each be 100 to 3,000 bp long and preferably 200 to 2,000 bp long, with a number of up to 20, for example, 1 to 20. A DNA contig formed by assembling rRNAs is a set of cloned DNA segments that overlap and are continuous. In this specification, overlapping and continuous data sets can be acquired and such data sets are called contigs.

Conventionally, for example, an entire genome is assembled de novo, segments with a length of 200 bp or less or 500 bp or less are abandoned and contigs which are each shorter than the full-length gene and have a length of thousand to hundreds of thousand bp are formed.

In the step of forming a contig, Rockopper, an RNA sequence assembly tailored program, may be used. The term "RNA sequence assembly customized program" refers to a program exclusively used to assemble DNA segments corresponding to RNA sequences.

An assembly algorithm for the RNA sequence assembly tailored program differs from conventional programs for de novo assembling genomes from NGS raw data (SPAdes, Velvet, SOAPdenovo, etc.) in the following aspects. Conventional programs for de novo genome assembly from NGS raw data (e.g., SPAdes, Velvet, SOAPdenovo, etc.) generally employ A de Bruijn graph algorithm. The de Bruijn graph algorithm produces results which greatly fluctuate depending on k-mer lengths and in which k-mer sizes for contigs similar to a full-length genome may be different from k-mer parameters for 16S rRNA full-length genes. Typically, de novo genome assembly uses, for example, a relatively high k-mer value (SPAdes=127) in order to increase assembly accuracy for repeat sequence parts.

The term "k-mer" typically refers to all the possible substrings of length k (subsequence) that are contained in a string. In computational genomics, k-mer refers to all possible subsequences (of length k) from a read obtained through DNA sequencing. The amount of k-mers possible given a string of length L is L-K+1, whilst the number of possible k-mers given n possibilities (4 (ACTG) in the case of DNA) is nk. K-mers are typically used during sequence assembly, but can also be used in sequence alignment.

The choice of the k-mer length or size has many different effects on the sequence assembly. These effects vary greatly between lower sized and larger sized k-mers as follows. A lower k-mer size will decrease the amount of edges stored in the graph, and as such, will help decrease the amount of space required to store DNA sequence. Having smaller sizes will increase the chance for all the k-mers to overlap, and as such, have the required subsequences in order to construct the de Bruijn graph. However, smaller sized k-mers also risk having many vertices in the graph leading into a single k-mer. Therefore, this will make the reconstruction of the genome more difficult as there is a higher level of path ambiguities due to the larger amount of vertices that will need to be traversed. Information is lost as the k-mers become smaller. Smaller k-mers also have the problem of not being able to resolve areas in the DNA where small microsatellites or repeats occur. This is because smaller k-mers will tend to sit entirely within the repeat region and it is therefore hard to determine the amount of repetition that has actually taken place.

In contrast, having larger sized k-mers will increase the amount of edges in the graph, which in turn, will increase the amount of memory needed to store the DNA sequence. By increasing the size of the k-mers, the number of vertices will also decrease, which will help with the construction of the genome as there will be fewer paths to traverse in the graph. Larger k-mers also run a higher risk of not having outward vertices from every k-mer. This is due to larger k-mers increasing the risk that it will not overlap with another k-mer by k-1. Therefore, this can lead to disjoints in the reads, and as such, can lead to a higher amount of smaller contigs. Larger k-mer sizes help alleviate the problem of small repeat regions. This is due to the fact that the k-mer will contain a balance of the repeat region and the adjoining DNA sequences that can help to resolve the amount of repetition in that particular area.

Because k-mer sizes have many different effects on the sequence assembly, the de novo genome assembly programs are run with relatively high k-mers (e.g., k-mer=127).

An RNA sequence assembly tailored program, for example, the RNA assembler Rockhopper is different from conventional de novo genome assembly programs in that the former additionally conducts filtering against the k-mer candidate groups to be assembled by employing a Burrow-wheeler indexing technique mainly used for mapping, together with de Bruijn graph algorithm. For example, the Rockhopper program uses k-mer=25 and performs an assembly process after calling of 16S rRNA. Thus, even in the case of a low k-mer value, the de novo genome assembly method does not suffer from the disadvantage of the low assembly accuracy attributed to repeated sequences.

**(d) Step of determining and excising an rDNA sequence region in the at least one rDNA contigs**

The method for obtaining ribosomal DNA having a completeness of 0.9 or higher in a single organism according to the present disclosure comprises the step of determining and excising an rDNA sequence region in at least one rDNA contig. This step allows the correction of at least one error selected from the group consisting of a nucleotide sequence error, a false gap, a tandem repeat error, a monopolymer error, and a single nucleotide polymorphism error, which occur in the step of merging segments.

In the step of excising the full-length rDNA sequence information from the rDNA contig and correcting an assembly error, only a 16S rRNA region is excised out of the read in order to exclude the added or repeated sequences resulting from the sequence assembly at terminal potions.

Examples of the assembly error include a nucleotide sequence error, a false gap error, a tandem repeat error, a monopolymer error, and a single nucleotide polymorphism (SNP) error, but are not limited thereto.

The step of calling full-length rDNA sequence information out of the rDNA contig and correcting an assembly error may be carried out using a program used in the step of calling rDNA sequence information out of NGS read. The program may be at least one selected from the group consisting of CMsearch, Hmmsearch, BLAST, and Usearch, but is not limited thereto.

Another embodiment of the present disclosure provides a method for identifying a microorganism, using a full-length ribosomal RNA nucleotide sequencing assay, the method comprising the steps of: obtaining full-length ribosomal sequence information by means of the sequencing assay; and comprising the full-length ribosomal RNA sequence information with a full-length ribosomal RNA taxonomic database to identify a microorganism.

The step of comparing the full-length ribosomal RNA sequence information with a full-length RNA taxonomic database may be conducted by, but not limited to, alignment with full-length RNA taxonomic database. The full-length RNA taxonomic database can be searched for and downloaded from sites including, but not limited to, the Nucleotide Database of the National Center for Biotechnology Information (NCBI), EzBioCloud (http://www.ezbiocloud.net/), Greengenes (http://greengenes.lbl.gov/), SILVA (https://www.arb-silva.de/), and RDP Database (https://rdp.cme.msu.edu/).

Alignment with a full-length ribosomal RNA taxonomic database may be carried out by searching for similar sequences within a well-known full-length ribosomal RNA sequence database for microorganism and the NCBI BLAST program and identifying a microorganism of interest with reference to a microorganism species which is found to have the most similar sequence to the searched sequences through Pairwise Alignment.

A further embodiment of the present disclosure provides a computer reading method for analyzing a full-length ribosomal RNA sequence by sequencing, the method comprising the step of obtaining full-length ribosomal RNA sequence information by means of a sequencing assay.

The computer reading method is intended to encompass the analysis of data on a computer with the aid of a computer program stored in a computer readable storage medium to execute the steps of the methods described herein, but is not limited thereto.

The methods and information described herein provide a computer program stored in a computer readable storage medium in order to execute the steps thereof. The computer program stored in a computer readable storage medium may be that associated with hardware. The computer program stored in a computer readable storage medium may be a program for executing the steps in a computer. In this regard, the steps may each be executed with one program or a combination of two or more programs. The program or software stored in a computer readable storage medium can be transferred to a computer device via any known transmission method including, for example, communication channels such as a telephone wire, the Internet, wireless connection, etc., and transportable media such as computer readable discs, flash drives, etc.

Yet another embodiment of the present disclosure provides a storage medium (or record medium) in which a computer readable program for executing steps of the method for obtaining full-length ribosomal RNA sequence information by means of a sequencing assay is stored.

A storage medium having the computer readable program recorded therein may include a computer storage medium and a communication medium. Computer storage media includes volatile and non-volatile, removable and non-removable media implemented in any method or technology for storage of information such as computer-readable instructions, data structures, program modules or other data. By way of example, and not limitation, computer storage media includes RAM, ROM, EEPROM, flash memory (e.g., USB memory, SD memory, SSD, CF memory, xD memory, and the like), magnetic discs, laser discs, or other memory technology, CD-ROM, digital video disc (DVD) or other optical disc storage, magnetic cassettes, magnetic tapes, magnetic disc storage or other magnetic storage devices, or any other medium which can be used to store the desired information and which can be accessed by the computer.

Communication media typically include computer readable instruction codes, data structures, program modules, or other data of a modulated data signal such as a carrier wave, or other transmission mechanism, and includes any information transmission medium. By way of example, and not limitation, the communication medium may be at least one selected from among wired media such as a wired network or direct-wired connection, and wireless media such as acoustic, RF, infrared and other wireless media.

At least one combination of the media may fall within the scope of the computer readable medium. A computer readable medium according to one embodiment of the present disclosure is illustrated in FIG. 2. By way of example, a computer system 500 may comprise at least one processor 510, at least one computer readable storage medium 530, and a memory 520.

While specific embodiments of and examples for the invention are described above for illustrative purposes, various equivalent modifications are possible within the scope of the invention, as those skilled in the relevant art will recognize.

A better understanding of the present disclosure may be obtained through the following examples that are set forth to illustrate, but are not to be construed as limiting the present disclosure.

### <COMPARATIVE EXAMPLE 1> Selection of 16S rRNA Sequence

For 215 microorganism samples, NGS (Next Generation Sequencing) raw data were obtained. NGS raw data were downloaded from the Sequence Read Archive (SRA) of the National Center for Biotechnology Information (NCBI) after search for bacteria genomes. Downloading is possible with the aid of SRA accession and SRA toolkit programs.

Using the SPAdes genome assembler program, assembly was made on the entire NGS raw data for the microorganisms to obtain DNA contigs.

16S rRNA sequences were called out of the obtained NA contigs, using the CMsearch program. <Parameter: E-value cutoff: 1e-20, Search space size: 700Mb>

### <EXAMPLE 1> Selection Method of 16S rRNA Sequence

In contrast to Comparative Example 1 in which NGS raw data was assembled, followed by calling all 16S rRNA sequences, Example 1 started with primarily calling 16S rRNA out of NGS raw data with the CMsearch program, followed by assembling the primarily called 16S rRNA and then secondarily calling all 16S rRNA sequences.

In brief, NGS (Next Generation Sequencing) raw data for 215 microorganism samples was obtained. NGS raw data was downloaded from the Sequence Read Archive (SRA) of the National Center for Biotechnology Information (NCBI) after a search for bacteria genomes. Downloading is possible with the aid of SRA accession and SRA toolkit programs.

Selecting 16S rRNA sequences out of the NGS raw data was conducted by calling all reads inclusive of 16S rRNA sequence information by means of the CMsearch program (primary calling of 16S rRNA sequences). <Parameter: E-value cutoff: 1e-20>

The assembly of the selected 16S rDNA sequences was conducted using the RNA assembler program Rockhopper. For the assembly, from a various number of cases in which segments were merged using k-mer through comparison between original sequences installed in the Rockhopper program and contigs into which segmented sequences has been merged, the cases of mergence of sequences which did not exist in the pre-segmentation sequence information when the merged sequences were compared to the pre-segmentation sequence information with respect to each mergence were extruded. <Parameter: mismatch allow: 0.5, minimum length: 1, minimum length de novo: 50, minimum count of k-mer:1>

After the assembly was carried out, entire 16S rRNA sequences were secondarily called in such a way that only 16S rRNA sequence information within reads was excised using the CMsearch program (secondarily calling of 16S rRNA sequences). <Parameter: E-value cutoff: 1e-20>1

### <EXPERIMENT EXAMPLE 1> Analysis of sample number with full-Length 16S rRNA

Out of 215 test samples, counts of samples from which full-length 16S rRNA was called through the methods of Comparative Example 1 and Example 1 were represented. Analysis results are given in Table 1 and FIG. 1. Briefly, 16S rRNA sequences were called using the methods of Comparative Example 1 and Example 1 in a total of 215 microorganism samples and determination was made to see whether entire sequences were called. Analysis results are given in Table 1 and FIG. 1.

[TABLE 1]

| Selection method | completeness | Number of samples(percentage) | Average completeness |
|---|---|---|---|
| Example 1 | 0.9 or higher | 210 samples (97.67%) | 0.9999 |
| Example 1 | Lower than 0.9 | 5 samples (2.32%) | 0.751 |
| Comparative Example 1 | 0.9 or higher | 196 samples (91.16%) | 0.997 |
| Comparative Example 1 | Lower than 0.9 | 19 samples (8.83%) | 0.706 |

FIG. 1 shows counts of samples from which full-length 16S rRNA were called through the methods of Comparative Example 1 and Example 1 out of 215 test samples. In FIG. 1, out of a total of 215 samples, samples from which full-length 16S rRNA was successfully called amounted to 210 for the method of Example 1, 196 for the method of Comparative Example 1, and 192 for both the methods of Comparative Example 1 and Example 1. Only the method of Example 1 was possible in 19 samples. When the method of Comparative Example 1 was applied to the 19 samples, full-length 16S rRNA were not called, but short segments were obtained (18 samples) or extraction itself was impossible (one sample).

**<EXPERIMENT EXAMPLE 2> Analysis of Selection Completeness of 16S rRNA Sequence**

For completeness comparison, 16S rRNA selected in Comparative Example 1 and Example 1 was analyzed for completeness as follows. Briefly, sequences that include regions of 16S rRNA covering up to the universal primer (27F, 1492R) region of 16S rRNA were collected from a full-length 16S rRNA sequence database to construct a complete 16S rRNA sequence database. A query sequence was matched and aligned with the database sequences to detect how many nucleotides of the reference sequence were deleted compared to the database sequences at front/behind sides to calculate the completeness.

Analysis results of the sequences of Example 1 are given in Table 2. As shown, when 16S rRNA sequences were called using the method of Example 1, 210 in a total of 215 samples were found to have a completeness of 0.9 or greater while a completeness less than 0.9 was calculated for five samples. Of the five samples, one was impossible for 16S rRNA calling. Among them, four samples had a completeness of 1 whilst one sample has a completeness of 0.805.

Analysis results of the sequences of Comparative Example 1 are given in Table 3. In a total of 215 microorganism samples, 196 were found to have a completeness of 0.9 or greater when 16S rRNA sequences were selected using the method of Comparative Example 1. A completeness of less than 0.9 was calculated for the remaining 19 samples. Of these samples, 18 were found to have a completeness of 1 when 16S rRNA sequences were called using the method of Example 1 while one was impossible for 16S rRNA selection.

[TABLE 2]

| Sample ID | Seq Length | Completeness | Similarity | Assign Taxon | NCBI |
|---|---|---|---|---|---|
| 10000078 | 1529 | 1 | 1 | Bacteroides ovatus | Bacteroides |
| 10000083 | 1540 | 1 | 0.994 | Salmonella enterica subsp. enteric a | Salmonella enterica subsp. enterica serovar Typhimurium str. LT2 |
| 10000084 | 1542 | 1 | 0.993 | Salmonella enterica subsp. enteric a | Salmonella enterica subsp. enterica serovar Typhimurium str. LT2 |
| 10000115 | 1552 | 1 | 1 | Bacillus anthracis | Bacillus anthracis |
| 10000136 | 1504 | 1 | 0.997 | Orientia tsutsugamushi | Orientia tsutsugamushi str. TA716 |
| 10000165 | 1542 | 1 | 0.998 | Citrobacter freundii | Citrobacter sp. AATXR |

(continued)

| Sample ID | Seq Length | Completeness | Similarity | Assign Taxon | NCBI |
|---|---|---|---|---|---|
| **10000210** | 1560 | 1 | 0.999 | Enterococcus faecium | Enterococcus faecium |
| **10000247** | 1503 | 1 | 03997 | Clostridium difficile | Peptoclostridium difficile 5.3 |
| **10000253** | 1503 | 1 | 0.997 | Clostridium difficile | Peptoclostridium difficile |
| **10000265** | 1552 | 1 | 1 | Bacillus anthracis | Bacillus anthracis |

[TABLE 3]

| Sampe ID | Seq Length | Complete ness | Similarity | Assign Taxon | NCBI |
|---|---|---|---|---|---|
| 10000078 | 968 | 0.667 | 1 | Bacteroides ovatus | Bacteroides |
| 10000083 | 1083 | 0.727 | 0.996 | Salmonella enterica subsp. enteric a | Salmonella enterica subsp. enterica serovar Typhimurium str. LT2 |
| 10000084 | 1001 | 0.671 | 0.998 | Salmonella enterica subsp. enteric a | Salmonella enterica subsp. enterica serovar Typhimurium str. LT2 |
| 10000115 | 1130 | 0.755 | 1 | Bacillus anthracis | Bacillus anthracis |
| 10000136 | 1137 | 0 | 0.588 | Orientia tsutsugamushi | Orientia tsutsugamushi str. TA716 |
| 10000165 | 1067 | 0.704 | 0.998 | Citrobacter freundii | Citrobacter sp. AATXR |
| 10000210 | 781 | 0.526 | 1 | Enterococcus hirae | Enterococcus faecium |
| 10000247 | 608 | 0.411 | 1 | Clostridium difficile | Peptoclostridium difficile 5.3 |
| 10000253 | 893 | 0.615 | 0.997 | Clostridium difficile | Peptoclostridium difficile |
| 10000265 | 1041 | 0.693 | 1 | Bacillus anthracis | Bacillus anthracis |

### <EXPERIMENTAL EXAMPLE 3> Analysis of sample ratio with selected Full-Length 16S rRNA

The ratio of samples in which full-length 16S rRNA was successfully selected only by the method of Example 1 was analyzed (raw data completeness =1).

Briefly, among the 16S rRNA sequences selected using the methods of Example 1 and Comparative Example 1, the samples from which full-length 16S rRNA sequences were called at a completeness of 1 by the method of Example 1 but at a completeness of less than 0.9 by the method of Comparative Example 1 were analyzed for ratio. The number of samples from which full-length 16S rRNA sequences could be called only by the method of Example 1, but not by the conventional method was 18, amounting about 8.37 % of the total samples. Of the 18 samples, ten samples were measured to have a completeness of less than 0.8 with an average completeness of 0.57, which accounts for remarkably low values in predominant samples except a few. Seven samples had a completeness of 0.7 or less, which was a level having a significant effect on microbial identification with 16S rRNA, and has an average completeness of as low as 0.51.

That is, only the method of Example 1 could call 16S rRNA sequences suitable for microbial identification from 18 of the 215 samples.

### <COMPARATIVE EXAMPLE 2> Sequences difference of 16S rRNA obtained by differing assembly program

In order to examine a difference in called 16S rRNA sequences depending on sequence assembly programs, the same method as is described in Example 1 was conducted for 10 microorganism samples shown in Table 1, with the exception that the primarily called 16S rRNA sequences were assembled using the DNA assembler SPAdes assembly program in Comparative Example 1, instead of the Rockhopper program in Example 1.

That is, 16S rRNA sequences for 10 samples were primarily called out of the NGS raw data in the same manner as in Example 1 and assembled using the genome DNA assembler SPAdes assembly program, followed by secondarily calling. With respect to the secondarily called 16S rRNA sequences, the number of the samples from which full-length 16S rRNA was called in the same manner as in Experiment Example 1 was examined. In brief, 16S rRNA sequences were called from a total of 215 microorganism samples, using the methods of Comparative Example 1 and Example 1 and examination was made to see whether full-length sequences were called. The analysis results are given in Table 4, below.

[TABLE 4]

| Sample ID | Example 1 (Rockhopper) | | Comparative Exampe 2 (SPAdes 3.7.0) | | | NCBI Taxon |
|---|---|---|---|---|---|---|
| | Seq Length | Assign Taxon | Seq Length | Similarity | Assign Taxon | |
| 10000078 | 1529 | B. ovatus | 1529 | 1 | B. ovatus | Bacteroides |
| 10000083 | 1540 | S. enterica subsp. enteric | 1083 | 0.996 | S. enterica subsp. enterica | Salmonella enterica subsp. enterica serovar Typhimurium str. LT2 |
| 10000084 | 1542 | S. enterica subsp. enteric | 1001 | 0.998 | S. enterica subsp. enteric | Salmonella enterica subsp. enterica serovar Typhimurium str. LT2 |
| 10000115 | 1552 | B. anthracis | 1552 | 1 | B. anthracis | Bacillus anthracis |
| 10000136 | 1504 | O. tsutsugamushi | 1504 | 0.997 | O. tsutsugamushi | Orientia tsutsugamushi str. TA716 |
| 10000165 | 1542 | C. freundii | 1542 | 0.998 | C. freundii | Citrobacter sp. AATXR |
| 10000210 | 1560 | E. faecium | 474 | 0.99 | E. faecium | Enterococcus faecium |
| 10000247 | 1503 | C. difficile | 608 | 1 | C. difficile | Peptoclostridium difficile 5.3 |
| 10000253 | 1503 | C. difficile | 893 | 0.997 | C. difficile | Peptoclostridium difficile |
| 10000265 | 1552 | B. anthracis | 1561 | 0.99 | E. faecalis ATCC 19433(T) | Bacillus anthracis |

As shown in Table 4, both the Rockhopper of Example 1 and the SPAdes of Comparative Example 2 successfully called full-length 16S rRNA sequences from four of a total of 10 samples (10000078, 10000115, 10000136, and 10000165). Five samples allowed full-length 16S rRNA sequences to be called only by the Rockhopper of Example 1, but failed the calling with the SPAdes of Comparative Example 2 (ID: 10000083, 10000084, 10000210. 10000247, 10000253). A full-length sequence was successfully called from one sample (ID 10000265) by means of the SPAdes of Comparative Example 2, but was found to be not a 16S rRNA. Microbial identification results were found be more accurate as obtained from the 16S rRNA constructed through the Rockhopper of Example 1 than the SPAdes of Comparative Example 2.

**<EXPERIMENTAL EXAMPLE 4> Identification of microorganism using Full-Length 16S rRNA Sequence**

In order to compare microorganism identification accuracy between the 16S rRNA calling methods of Example 1 and Comparative Example 1, microorganisms were identified using the 16S rRNA sequences that were respectively called in Example 1 and Comparative Example 1 from 10 microorganism samples showed in Table 2, as follows.

Briefly, a 16S rRNA sequence database for a known microorganism and the NCBI BLAST program were used to search for the full-length 16S rRNA sequences called using the method of Example 1. The resulting similar sequences were subjected to Pairwise Alignment to determine the query microorganisms to be species of the most similarity. Experiment results are summarized in Tables 2 and 3.

As shown in Tables 2 and 3, ID 10000210 was identified as a species different from the accurate microorganism enrolled in the NCBI when the 16S rRNA called by the method of Comparative Example 1 was used, but as the very species enrolled in the NCBI when the 16S rRNA was called by the method of Example 1.

**<EXPERIMENTAL EXAMPLE 5> Identification of microorganism using Full-Length 16S rRNA Sequence**

In order to compare microorganism identification accuracy between the 16S rRNA calling methods of Example 1 and Comparative Example 2, microorganisms were identified in a similar manner to that of Experiment Example 4, using the 16S rRNA sequences that were respectively called in Example 1 and Comparative Example 1 from 10 microorganism samples shown in Table 2. The experiment results are given in Table 4.

For nine samples, as shown in Table 4, the identification results obtained by the SPAdes assembly program of Comparative Example 2 were identical to those obtained by the RNA assembler Rockhopper program of Example 1. However, the remaining one sample (full-length 16S rRNA called in both the SPAdes assembly program and the RNA assembler Rockhopper program) was more accurately identified by the Rockhopper program than the SPAdes program (ID: 10000265).

**<EXPERIMENTAL EXAMPLE 6> Identification of 16S rRNA segment in sequences discarded upon assembly**

6-1: Analysis Method

In order to examine whether a 16S rRNA segment exists in the sequences lost during conventional assembly, only the reads discarded after assembly were subjected to 16S rRNA calling and analyzed for a degree of loss of 16S rRNA. When assembly was conducted for NGS raw data according to the method of Comparative Example 1, short sequences are not regarded to be assembled with other sequence due to a sequencing error occurring during the production of NGS raw data. Thus, the short sequences (200 bp or less) are dropped during an assembly procedure in order to improve the quality of assembly results for full-length genomes. Therefore, when a 16S rRNA sequence is contained in the short sequences (200 bp or less) dropped during assembly, a part of 16S rRNA sequence information is lost. However, the method of the present disclosure is expected to not lose 16S rRNA sequence information, unlike the method of Comparative Example 1. To verify this expectation, sequences dropped during assembly were analyzed as follows.

With regard to 10 microorganism samples listed in Table 2 (ID 10000078, 10000083, 10000084, 10000115, 10000136, 10000165, 10000210, 10000247, 10000253, and 10000265), 16S rRNA sequences were called by means of the CMsearch program out of the reads [(paired(2), unpaired(1)] dropped after assembly in the analysis method of Comparative Example 1. The resulting sequences were compared to the 16S rRNA sequences called in Comparative Example 1 to analyze the ratio of the drop read to the entire reads for called 16S rRNA sequences.

For NGS sequencing, there are two runs: a paired end run that sequences DNA from both ends of a DNA segment; and a single end run that sequences DNA only from one end. The number of files having drop reads generated after assembly are determined according to NGS sequencing runs. When conducted in a paired end run, NGS sequencing produces a total number of three files composed of two files including reads generated in pairs (PI, P2) and one file generated in a unpaired mode (U). In contrast, NGS sequencing in a single-end run has one file (PI) because pairs are not formed. For ID 10000083 sample, only P1 was generated with single-end sequencing.

6-2: Analysis for rates of 16S rRNA sequence in drop reads when a full-length sequence is not called by the method of Comparative Example 1

In the case where a full-length sequence was not called by the method of Comparative Example 1, rates of 16S rRNA sequences in the dropped reads were calculated using the following formula and are given in Table 5, below. Table 5 shows rates of 16S rRNA sequences in each file.

[Mathematical Formula 2]

$$\text{The rate of dropped 16S reads(\%)} = \frac{\text{number of dropped 16S reads}}{\text{total number of dropped reads}} \times 100$$

[TABLE 5]

| Sample ID | Rate(16S reads: drop reads) | | | completeness |
|---|---|---|---|---|
| | P1(%) | P2(%) | U(%) | |
| 10000078 | 1.51 | 1.82 | 4.10 | 0.667 |
| 10000083 | 12.47 | • | • | 0.727 |
| 10000084 | 0.79 | 0.80 | 0.0 | 0.671 |
| 10000115 | 2.73 | 7.69 | 10.52 | 0.755 |
| 10000136 | 8.04 | 4.77 | 0.0 | 0 |
| 10000165 | 2.38 | 4.88 | 0.0 | 0.704 |
| 10000210 | 1.25 | 1.95 | 3.82 | 0.526 |
| 10000247 | 2.75 | 3.91 | 4.05 | 0.411 |
| 10000253 | 2.25 | 3.60 | 0.0 | 0.615 |
| 10000265 | 2.98 | 6.45 | 0.0 | 0.693 |

As shown in Table 5, when the 16S rRNA sequences were called after assembly as in Comparative Example 1, the drop reads did not contain any of 16S rRNA sequences or contained 16S rRNA sequences at a small portion.

6-3: Analysis for rates of 16S rRNA sequence in drop reads when a full-length sequence is called by the method of Comparative Example 1

In the case where a full-length sequence was called by the method of Comparative Example 1, rates of 16S rRNA sequences in the dropped reads were calculated using Mathematical Formula 2 and are given in Table 6, below.

[TABLE 6]

| Sample ID | Rate(16S reads: drop reads) | | | completeness |
|---|---|---|---|---|
| | P1(%) | P2(%) | U(%) | |
| 10000068 | 1.10 | 0.69 | 2.56 | 1 |
| 10000069 | 0.99 | 3.24 | 0.0 | 1 |
| 10000070 | 0.48 | 9.61 | 6.66 | 1 |
| 10000071 | 14.96 | 2.50 | 7.01 | 1 |
| 10000072 | 2.14 | 1.76 | 5.88 | 1 |
| 10000073 | 0.40 | 0.52 | 6.66 | 1 |
| 10000074 | 0.25 | 1.04 | 2.43 | 1 |
| 10000075 | 0.25 | 0.34 | 1.21 | 1 |
| 10000076 | 0.41 | 0.65 | 0 | 1 |
| 10000077 | 0.54 | 0.97 | 5.26 | 1 |

As shown in Table 6, when full-length 16S rRNA sequences were called after assembly by the method of Comparative Example 1, a certain amount of 16S rRNA sequences were found in the drop reads which were produced after assembly for NGS raw data of a total of 10 microorganism samples. Because these sequences may be necessary for calling full-length 16S rRNA sequences, a loss may be determined to occur in the called 16S rRNA.
Among 10 samples, as shown in Table 6, six samples including ID 10000068, 10000073, 10000074, 10000075, 10000076, and 10000077 had remarkably low rates of dropped 16S rRNA reads. Exceptively, four samples including ID 10000069, 10000070, 10000071, and 10000072 exhibited high rates of dropped reads. This may be the case where full-length 16S rRNA sequences can be called with the remaining 16S rRNA reads although the dropped reads are not used.

In comparing the results in Tables 5 and 6, higher rates of 16S rRNA sequences in dropped reads were detected in the samples, except for a few samples, when full-length 16S rRNA sequences were not called by the method of Comparative Example 1.

**Claims**

1. A method for obtaining a ribosomal DNA of a single organism, having a completeness of 0.90 or higher, comprising:

   obtaining genome NGS reads including ribosomal DNA (rDNA) sequence information of the single organism by next-generation sequencing (NGS),
   selecting the reads including rDNA sequence information from the genome NGS reads
   segmenting the selected reads and merging the segments for assembly to form one or more rDNA contigs
   deteriming and excising an rDNA sequence region in the one or more rDNA contigs to obtain the rDNA sequence region.

2. The method of claim 1, wherein the step of selecting the read includes selecting a read which includes rDNA sequence information and is in a length of a K-mer length or longer enough to be used for assembly.

3. The method of claim 1, wherein the step of selecting the read includes selecting a read having an E-value of $10^{-20}$ or less by using sequence pattern information obtained by alignment with a known full-length rRNA sequence.

4. The method of claim 3, wherein the sequence pattern information obtained by alignment with a known full-length rRNA sequence is a sequence pattern information obtained by utilizing hidden Markov model (HMM) or covariance model (CM).

5. The method of claim 1, wherein the step of forming DNA contigs is carried out by using an RNA assembly program.

6. The method of claim 1, wherein the segmenting is performed to produce the reads having a K-mer value of 7 to 36.

7. The method of claim 1, wherein the step of merging segments to form rDNA contigs includes a merging process of segments and a verifying process in which the merged segments are aligned with the selected reads to examine whether the merged segments are the sequence regions within the reads.

8. The method of claim 7, wherein the merging process and the verifying process are repeated two or more times.

9. The method of claim 1, wherein the step of determining an rDNA sequence region in the rDNA contigs includes determining an rDNA sequence region having an E-value of $10^{-20}$ or less by using sequence pattern information obtained by alignment with a known full-length rRNA sequence.

10. The method of claim 1, wherein the step of determining and excising an rDNA sequence makes the correction of at least one selected from the group consisting of a nucleotide sequence error, a false gap, a tandem repeat error, a monopolymer error, and a single nucleotide polymorphism error, which are generated in a merging process of segments.

11. The method of claim 1, wherein the step of selecting a read or the step of determining an rDNA sequence region in an rDNA contig is carried out using CMsearch, Hmmsearch, BLAST, or Usearch program.

12. The method of claim 1, wherein the length of obtained ribosomal RNA ranges from 1,300 bp to 1,800 bp.

13. The method of claim 1, wherein the rDNA is 16S rDNA, 23S rDNA, or 5S rDNA.

14. A method for identifying a microorganism using an rRNA sequence information analysis, comprising:

   obtaining a ribosomal DNA of a single organism with a completeness of 0.90 or higher according to the method of any one of claims 1 to 13; and
   identifying a microorganism by comparing the sequence information of the obtained rRNA with a taxonomic database of rRNA.

**15.** A computer program, stored in a computer readable storage medium, for executing at least one step of the method according to any one of claims 1 to 13.

**16.** A computer storage medium in which a computer readable program for executing at least one step of the method according to any one of claims 1 to 13 is stored.

FIG. 1

FIG. 2

# EP 3 511 847 A1

<table>
<tr><td colspan="2">INTERNATIONAL SEARCH REPORT</td><td>International application No.<br><br>**PCT/KR2017/015377**</td></tr>
</table>

**A. CLASSIFICATION OF SUBJECT MATTER**

*G06F 19/24(2011.01)i, G06F 19/28(2011.01)i, G06F 19/22(2011.01)i*

According to International Patent Classification (IPC) or to both national classification and IPC

**B. FIELDS SEARCHED**

Minimum documentation searched (classification system followed by classification symbols)
G06F 19/24; C07H 21/04; G06F 19/18; C07H 21/02; G06F 19/22; C12Q 1/68; G06F 19/28

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: ribosome DNA, mono-organism genome read, next-generation sequencing, contig, assembly

**C. DOCUMENTS CONSIDERED TO BE RELEVANT**

| Category* | Citation of document, with indication, where appropriate, of the relevant passages | Relevant to claim No. |
|---|---|---|
| A | BURKE, Catherine M. et al., "A Method for High Precision Sequencing of Near Full-length 16S rRNA Genes on an Illumina MiSeq", Peer J, 20 September 2016, vol. 4, e2492, inner pages 1-20<br>See abstract; and inner pages 2-9. | 1-16 |
| A | KR 10-1447593 B1 (SEOUL NATIONAL UNIVERSITY R&DB FOUNDATION) 07 October 2014<br>See abstract; and claims 1-8. | 1-16 |
| A | US 2004-0097718 A1 (PEARSON, James D. et al.) 20 May 2004<br>See the entire document. | 1-16 |
| A | WO 2015-177570 A1 (UNIVERSITY OF TECHNOLOGY, SYDNEY et al.) 26 November 2015<br>See the entire document. | 1-16 |
| A | YUAN, Cheng et al., "Reconstructing 16S rRNA Genes in Metagenomic Data", Bioinformatics, 2015, vol. 31, no. 12, pages i35-i43<br>See the entire document. | 1-16 |
| PX | KR 10-1798229 B1 (CHUNLAB, INC.) 12 December 2017<br>See abstract; claims 1-4, 6, 8-12; paragraphs [0065]-[0123]; and figure 1. | 1-16 |

☐ Further documents are listed in the continuation of Box C.   ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 APRIL 2018 (06.04.2018) | **06 APRIL 2018 (06.04.2018)** |
| Name and mailing address of the ISA/KR<br>Korean Intellectual Property Office<br>Government Complex-Daejeon, 189 Seonsa-ro, Daejeon 302-701, Republic of Korea<br>Facsimile No. +82-42-481-8578 | Authorized officer<br><br>Telephone No. |

Form PCT/ISA/210 (second sheet) (January 2015)

**INTERNATIONAL SEARCH REPORT**
Information on patent family members

International application No.

**PCT/KR2017/015377**

| Patent document cited in search report | Publication date | Patent family member | Publication date |
|---|---|---|---|
| KR 10-1447593 B1 | 07/10/2014 | WO 2015-102226 A1 | 09/07/2015 |
| US 2004-0097718 A1 | 20/05/2004 | AU 8286001 A<br>EP 1399562 A2<br>WO 02-08265 A2<br>WO 2002-008265 A3 | 05/02/2002<br>24/03/2004<br>31/01/2002<br>08/01/2004 |
| WO 2015-177570 A1 | 26/11/2015 | AU 2015-263055 A1<br>CA 2949925 A1<br>CN 107002120 A<br>EP 3146070 A1<br>JP 2017-517282 A<br>KR 10-2017-0012390 A<br>SG 11201609754 A<br>US 2017-0183724 A1 | 26/11/2015<br>26/11/2015<br>01/08/2017<br>29/03/2017<br>29/06/2017<br>02/02/2017<br>29/12/2016<br>29/06/2017 |
| KR 10-1798229 B1 | 12/12/2017 | NONE | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MARGULIES et al.** *Nature,* 2005, vol. 437, 376-380
- **HARRIS et al.** *Science,* 2008, vol. 320, 106-109
- **SONI ; MELLER.** *Clin Chem,* 2007, vol. 53, 1996-2001
- **DEAR.** *Brief Funct Genomic Proteomic,* 2003, vol. 1, 397-416